# EUROPEAN PATENT APPLICATION

(11) **EP 3 202 330 A1**
(43) Date of publication of application: **09.08.2017**
(21) Application number: 15846924.7
(22) Date of filing: 05.10.2015
(51) Int. Cl.: A61B 10/00

(54) **BOWEL MOVEMENT PREDICTION DEVICE AND BOWEL MOVEMENT PREDICTION METHOD**

(30) Priority: 03.10.2014 JP 2014204745
(71) Applicant: Triple W Japan K.K., Tokyo 1500031 (JP)
(72) Inventor: NAKANISHI, Atsushi, Tokyo 1500031 (JP); KUZURYU, Yuichiro, Tokyo 1500031 (JP)
(74) Representative: advotec.
(86) International application number: PCT/JP2015/078191
(87) International publication number: WO 2016/052747

(57) **Abstract**

The bowel movement prediction device 100 includes an ultrasonic sensor 1 that detects a width of a rectum and a determiner 52 that determines a bowel movement timing based on an output of the ultrasonic sensor 1.

## Description

### TECHNICAL FIELD

The technique disclosed here relates to a bowel movement prediction device and a bowel movement prediction method.

### BACKGROUND ART

A device for predicting bowel movement has been known to date. For example, a device disclosed in Patent Document 1 detects a defecation desire of a subject by noninvasively detecting, from outside the body of the subject, that a detection target previously taken in the subject reaches the rectum.

### CITATION LIST

### PATENT DOCUMENT

PATENT DOCUMENT 1: Japanese Patent Publication No. 2009-247690

### SUMMARY OF THE INVENTION

### TECHNICAL PROBLEM

In the technique disclosed in Patent Document 1, however, a subject needs to swallow a foreign substance such as a detection target. Thus, this technique involves pain and inconvenience. In cases where the subject is a person requiring care, he or she might not be able to swallow such a foreign substance smoothly.

It is therefore an object of the technique disclosed here to predict a bowel movement timing without a feel of pain or inconvenience of a subject.

### SOLUTION TO THE PROBLEM

A bowel movement prediction device disclosed here includes: a sensor that detects a width of a rectum; and a determiner that detects a bowel movement timing based on an output of the sensor.

A bowel movement prediction method disclosed here includes the steps of: detecting a width of a rectum; and determining a bowel movement timing based on the detected width of the rectum.

### ADVANTAGES OF THE INVENTION

The bowel movement prediction device can predict a bowel movement timing without a feel of pain or inconvenience of a subject.

The bowel movement prediction method can predict a bowel movement timing without a feel of pain or inconvenience of a subject.

### BRIEF DESCRIPTION OF THE DRAWINGS

[FIG. 1] FIG. 1 is a block diagram of a bowel movement prediction device.
[FIG. 2] FIG. 2 illustrates how the bowel movement prediction device is used.
[FIG. 3] FIG. 3 is a flowchart depicting determination of a bowel movement timing.
[FIG. 4] FIG. 4 is an example waveform of a received signal subjected to signal processing by a signal processor.

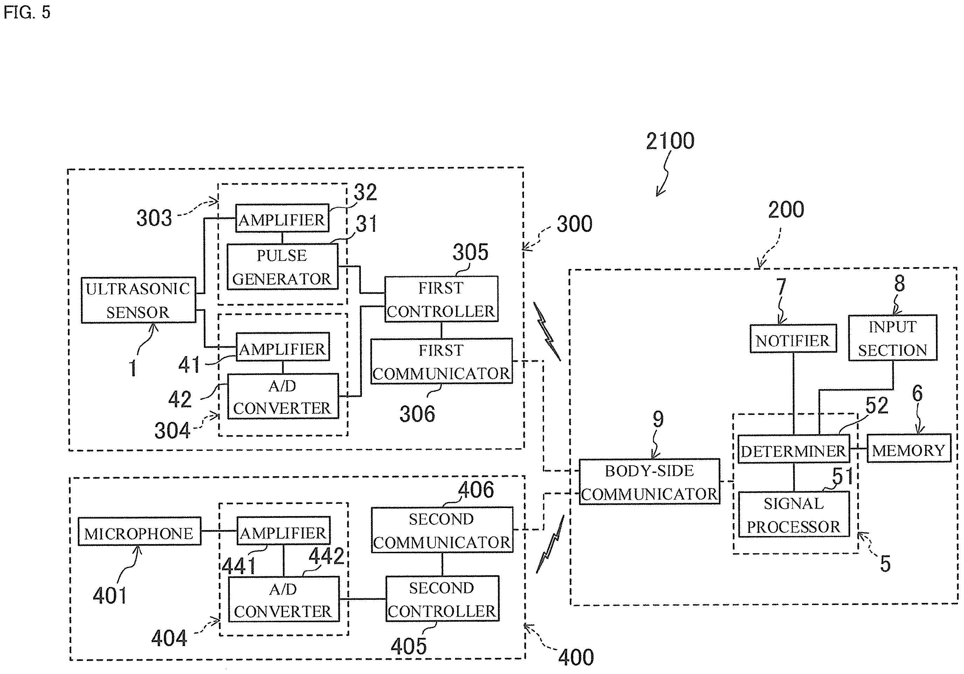

[FIG. 5] FIG. 5 is a block diagram of a bowel movement prediction device including a bowel sound sensor.

### DESCRIPTION OF EMBODIMENTS

An exemplary embodiment will be specifically described hereinafter with reference to the drawings.

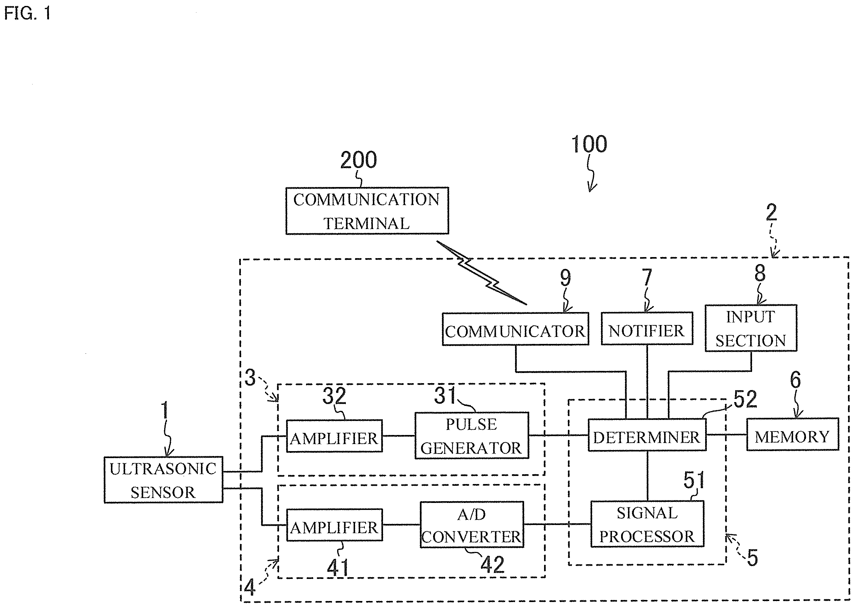

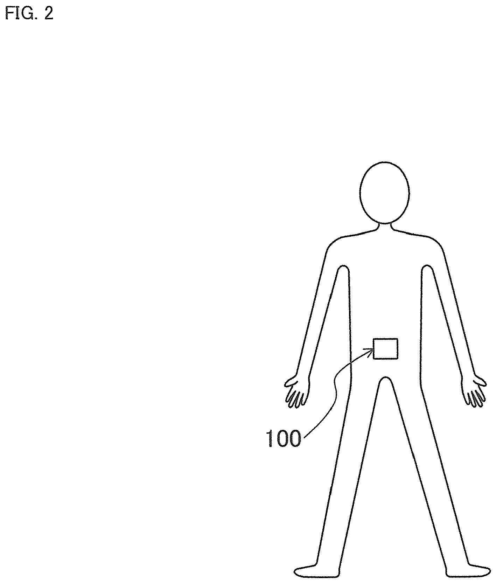

FIG. 1 is a block diagram of a bowel movement prediction device 100. FIG. 2 illustrates how the bowel movement prediction device 100 is used.

The bowel movement prediction device 100 is used for predicting a bowel movement timing of a subject. Examples of the subject include those whose require care, such as elderly people and physically disabled people, and those who do not require care but have difficulty in moving and need time for going to the lavatory. The bowel movement prediction device 100 includes an ultrasonic sensor 1 that detects the width of the rectum and a device body 2 that controls the ultrasonic sensor 1. The subject wears the bowel movement prediction device 100. Specifically, at least the ultrasonic sensor 1 is disposed on the skin of the subject at a location corresponding to the rectum (e.g., lower abdomen). The ultrasonic sensor 1 and the device body 2 are integrally provided.

The ultrasonic sensor 1 transmits and receives ultrasonic waves. Specifically, the ultrasonic sensor 1 includes a transducer constituted by a piezoelectric element. The ultrasonic sensor 1 generates ultrasonic waves by generating vibrations in accordance with a driving voltage, and when receiving ultrasonic waves, generates an electrical signal in accordance with the vibrations thereof. The ultrasonic sensor 1 is an example of a sensor.

The device body 2 includes a transmitter 3 that outputs the driving voltage to the ultrasonic sensor 1, a receiver 4 that receives the electrical signal from the ultrasonic sensor 1, a controller 5 that controls the entire bowel movement prediction device 100 and determines a bowel movement timing, a memory 6, a notifier 7 that issues a notification of arrival of a bowel movement timing, an input section 8 for inputting occurrence of actual bowel movement, and a communicator 9 that communicates with an external device.

The transmitter 3 supplies the driving voltage to the ultrasonic sensor 1. The transmitter 3 includes a pulse generator 31 and an amplifier 32. The pulse generator 31 generates a pulse signal having a predetermined duration of time and a predetermined voltage value. The amplifier 32 amplifies the pulse signal from the pulse generator 31, and outputs the amplified pulse signal to the ultrasonic sensor 1 as the driving voltage.

The receiver 4 receives the electrical signal from the ultrasonic sensor 1. The receiver 4 includes an amplifier 41 and an analog-to-digital (A/D) converter 42. The amplifier 41 amplifies a signal received from the ultrasonic sensor 1 and outputs the amplified signal to the A/D converter 42. The A/D converter 42 performs A/D conversion on the signal received from amplifier 41 and outputs the resulting signal to a signal processor 5.

The communicator 9 communicates with an external communication terminal. In the bowel movement prediction device 100, an external communication terminal for communication can be registered, and the memory 6 stores information on this communication terminal. That is, a user registers a communication terminal 200 in the bowel movement prediction device 100 beforehand. In this manner, communication between the bowel movement prediction device 100 and the communication terminal 200 can be established. For example, a communication terminal 200 carried by a caregiver is registered in the bowel movement prediction device 100 beforehand. In cases where the subject can go to the lavatory by himself or herself, the communication terminal 200 of the subject is registered beforehand. The number of communication terminals 200 is not limited to one, and a plurality of communication terminals 200 (e.g., a communication terminal of a caregiver and a communication terminal of a person requiring care) may be registered.

The controller 5 includes one or more processors. The controller 5 controls the transmitter 3 and causes the transmitter 3 to output the driving voltage to the ultrasonic sensor 1, and determines a bowel movement timing based on a signal received from the receiver 4. The controller 5 includes a signal processor 51 that performs signal processing on the signal received from the receiver 4 and a determiner 52 that controls the transmitter 3 and determines a bowel movement timing.

The signal processor 51 performs signal processing such as averaging on the signal received from the receiver 4, and outputs the resulting signal to the determiner 52.

The determiner 52 issues an instruction for generating the pulse signal to the pulse generator 31 of the transmitter 3, and determines a bowel movement timing based on the received signal input from the signal processor 51. The transmitter 3 may be controlled by a section except the determiner 52.

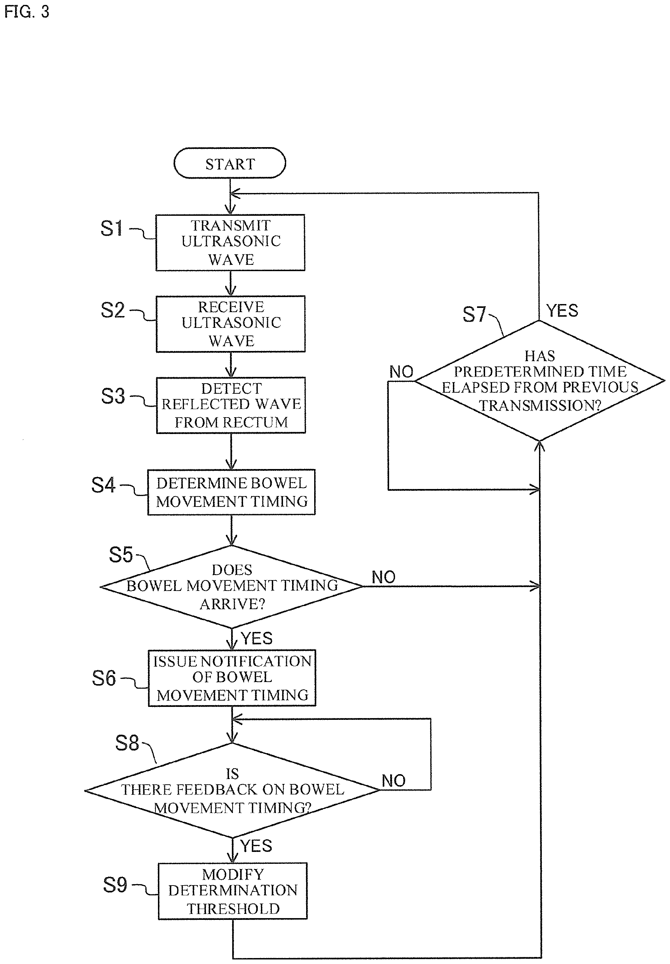

A process of the determiner 52 will now be described with reference to the flowchart of FIG. 3.

First, the determiner 52 outputs an instruction for transmitting ultrasonic waves (step S1). Specifically, the determiner 52 outputs an instruction for generating a pulse signal to the transmitter 3. This generation instruction serves as a trigger of a determination process for a bowel movement timing.

Thereafter, when the ultrasonic sensor 1 receives ultrasonic waves (i.e., when a received signal is input to the signal processor 51), the determiner 52 detects, based on the received signal, a reflected wave from the anterior wall (wall toward the abdomen) of the rectum and a reflected wave from the posterior wall (wall toward the back) of the rectum (step S3).

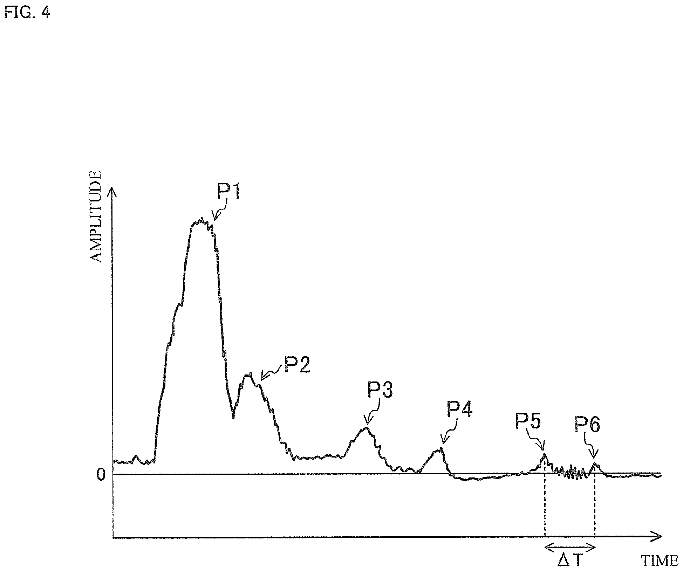

FIG. 4 illustrates an example waveform of a received signal subjected to signal processing by the signal processor 51. The first large peak P1 in FIG. 4 corresponds to noise in transmission, and is a so-called dead zone. The second peak P2 corresponds to a reflected wave from the skin surface. The third peak P3 corresponds to a reflected wave from the anterior wall (wall toward the abdomen) of the urinary bladder. The fourth peak P4 corresponds to a reflected wave from the posterior wall (wall toward the back) of the urinary bladder. The fifth peak P5 corresponds to a reflected wave from the anterior wall of the rectum. The sixth peak P6 corresponds to a reflected wave from the posterior wall of the rectum. The determiner 52 detects a time difference ΔT between the fifth peak P5 and the sixth peak P6 as a determination value. The determiner 52 can define, as the time difference ΔT between the peaks P5 and P6, a time difference between tops of the peaks P5 and P6 or a time difference between zero crossing points of the peaks P5 and P6.

Subsequently, the determiner 52 determines a bowel movement timing (step S4). Specifically, the determiner 52 determines whether the time difference ΔT between two reflected waves is greater than or equal to a predetermined determination threshold or not. The time difference ΔT between the two reflected waves corresponds to the width of the rectum. The determination threshold is a time difference between two reflected waves corresponding to the width of the rectum when the bowel movement timing arrives. The bowel movement timing may be set at an arbitrary time (e.g., a time immediately before bowel movement or a time 10 minutes before bowel movement). A determination threshold corresponding to this time is set in the memory 7 beforehand. That is, the determiner 52 determines whether the width of the rectum reaches a width assumed to correspond to the bowel movement timing or not.

The determiner 52 reads the determination threshold from the memory 6, and compares the readout determination threshold with the time difference ΔT that is a determination value (step S5). If the determination value is greater than or equal to the determination threshold (i.e., YES), the determiner 52 determines that the bowel movement timing arrives and issues a notification of arrival of the bowel movement timing (step S6). Specifically, the determiner 52 causes the notifier 7 to operate and issues a notification of arrival of the bowel movement timing to a registered communication terminal 200 through the communicator 9. The notifier 7 is, for example, a vibrator. Vibrations of the vibrator notify the subject of arrival of the bowel movement timing. In this manner, the subject is previously notified of a defecation desire that is to arise in near future, and thus, can prepare for going to the lavatory.

The communication terminal 200 may include a downloaded application dedicated to the bowel movement prediction device 100 so that communication with the bowel movement prediction device 100 and an operation of the bowel movement prediction device 100 can be performed. When the communication terminal 200 receives a signal indicating arrival of the bowel movement timing from the bowel movement prediction device 100, the communication terminal 200 notifies a user carrying the communication terminal 200 of arrival of the bowel movement timing by, for example, displaying a notification of arrival of the bowel movement timing on a display. In this manner, the user carrying the communication terminal 200 is notified of arrival of the bowel movement timing so that guidance to the lavatory for the subject can be promoted.

On the other hand, if the determination value is less than the determination threshold (i.e., NO), the determiner 52 determines whether a predetermined time has elapsed or not from the previous transmission of ultrasonic waves (step S7). If the predetermined time has elapsed, the determiner 52 returns to step S1 and transmits ultrasonic waves. On the other hand, if the predetermined time has not elapsed, the determiner 52 waits for a lapse of the predetermined time, and after the lapse of the predetermined time, returns to step S1. In this manner, the determiner 52 transmits ultrasonic waves in predetermined time intervals, and regularly performs determination of a bowel movement timing.

Every time the determiner 52 regularly performs determination of a bowel movement timing (a series of processes from an instruction for generating a pulse signal to determination), the determiner 52 stores the determination value in the memory 6. In other words, the memory 6 stores a chronological change of determination values.

After having issued a notification of arrival of a bowel movement timing, the determiner 52 waits for a report on occurrence of actual bowel movement (step S8). The device body 2 includes the input section 8. When bowel movement actually finishes, the input section 8 is operated by the subject or a third party such as a caregiver. The input section 8 is, for example, a push button. The report on occurrence of actual bowel movement can also be input from the communication terminal 200 to the bowel movement prediction device 100. When actual bowel movement finishes, a person carrying the communication terminal 200 operates the communication terminal 200 to transmit the report on occurrence of actual bowel movement to the bowel movement prediction device 100.

When receiving a signal from the input section 8 or the report on occurrence of actual bowel movement from the communication terminal 200, the determiner 52 determines that actual bowel movement occurred, and modifies the determination threshold (step S9). Specifically, based on a time from when a notification of arrival of a bowel movement timing is issued to when an input of a report on occurrence of actual bowel movement is received from the input section 8 or the communication terminal 200, the determiner 52 determines whether the notification of arrival of the bowel movement timing is issued at an appropriate time or not. A time required from when the width of the rectum reaches a width corresponding to the determination threshold to when bowel movement occurs substantially coincides with the time from issuance of the notification to the report on bowel movement. In view of this, as long as the time difference between the time from issuance of the notification to the report on bowel movement and the time to bowel movement assumed as a bowel movement timing is within a predetermined range, the determiner 52 does not change the determination threshold. In such a case, since the determiner 52 issues the notification of the bowel movement timing at an appropriate time, the determiner 52 does not modify the determination threshold. On the other hand, if the time from issuance of the notification to the report on bowel movement is shorter than the assumed time to bowel movement below the predetermined range, the determiner 52 reduces the determination threshold by a predetermined amount. If the time from issuance of the notification to the report on bowel movement is longer than the assumed time to bowel movement beyond the predetermined range, the determiner 52 increases the determination threshold by a predetermined amount. In such cases, since the expected time necessary to bowel movement is different from an actual time necessary to bowel movement, the determiner 52 modifies the determination threshold so that the time difference decreases. The determiner 52 overwrites the modified determination threshold on the memory 6. The determiner 52 uses the modified determination threshold for subsequent determinations of bowel movement timings.

The width of the rectum varies among individuals. Similarly, the width of the rectum immediately before bowel movement also varies among individuals. On the occurrence of actual bowel movement, the determination threshold is modified based on feedback so that a bowel movement timing can be accurately determined with a determination threshold depending on a subject.

When having finished modification of the determination threshold, the determiner 52 proceeds to step S7. That is, step S7 has been described as a process after determination of a bowel movement timing in a case where a bowel movement timing has not arrived yet, in a case where the bowel movement timing has arrived, the determiner 52 also performs the process of step S7 eventually. In this manner, irrespective of whether a bowel movement timing arrives or not, the determiner 52 transmits ultrasonic waves at predetermined time intervals, and regularly performs determination of a bowel movement timing.

As described above, the bowel movement prediction device 100 includes the ultrasonic sensor 1 that detects the width of the rectum and the determiner 52 that determines a bowel movement timing based on an output of the ultrasonic sensor 1.

In other words, the bowel movement prediction method described above includes the steps of detecting the width of the rectum and determining a bowel movement timing based on the detected width of the rectum.

In this configuration, the ultrasonic sensor 1 detects the width of the rectum. Immediately before bowel movement, fecal matter is accumulated in the rectum so that the rectum has a large width. That is, the width of the rectum indicates whether fecal matter is present in the rectum or not. In view of this, the determiner 52 determines a bowel movement timing from the width of the rectum based on an output of the ultrasonic sensor 1. For example, it can be determined that bowel movement will arrive soon if the width of the rectum exceeds a predetermined determination threshold.

The bowel movement prediction device 100 further includes a notifier 7 that issues a notification of arrival of a bowel movement timing. The determiner 52 causes the notifier 7 to operate when the determiner 52 determines that a bowel movement timing arrives.

With this configuration, when the determiner 52 determines that a bowel movement timing arrives, the notifier 7 issues a notification of this arrival to an external device. In this manner, the subject wearing the ultrasonic sensor 1 or another third party can be notified of a bowel movement timing. For example, in cases where the subject is a person who has difficulty in moving and needs time for going to the lavatory, the subject can be previously notified of bowel movement before he or she feels a defecation desire by setting the time of issuing the notification of a bowel movement timing earlier. In this manner, the subject is encouraged to prepare for going to the lavatory relatively early. In cases where the subject is a person requiring care, the notification of bowel movement can be previously issued even before the caregiver feels a defecation desire. In this manner, a caregiver can guide the person requiring care to the lavatory with a sufficient margin of time.

In addition, the determiner 52 is configured to determine that a bowel movement timing arrives if the width of the rectum is greater than or equal to a predetermined determination threshold, accept feedback on actual bowel movement after the determination of the bowel movement timing, and modify the determination threshold.

With this configuration, the determiner 52 determines a bowel movement timing based on the width of the rectum. However, since the width of the rectum varies among individuals, a determination threshold of the width of the rectum for use in determination of arrival of a bowel movement timing also varies among individuals. The determiner 52 accepts feedback on actual bowel movement after the determination of determination timing, and modifies the determination threshold. In this manner, the determination threshold is repeatedly modified depending on the subject. Consequently, a determination accuracy in determining a bowel movement timing by the determiner 52 can be enhanced.

Specifically, the determiner 52 is configured to accept an input indicating occurrence of actual bowel movement, and modifies the determination threshold based on a time from when the notification of a bowel movement timing is issued to when the input indicating occurrence of actual bowel movement is accepted.

In this manner, the determination threshold can be modified based on actual bowel movement.

### Other Embodiments

In the foregoing section, the embodiment has been described as an example of the technique disclosed in the present application. The technique disclosed here, however, is not limited to this embodiment, and is applicable to other embodiments obtained by changes, replacements, additions, and/or omissions as necessary. Components described in the above embodiment may be combined as a new exemplary embodiment. Components provided in the accompanying drawings and the detailed description can include components unnecessary for solving problems as well as components necessary for solving problems in order to exemplify the technique. Therefore, it should not be concluded that such unnecessary components are necessary only because these unnecessary components are included in the accompanying drawings or the detailed description.

The above embodiment may be configured as described below.

The sensor for detecting the width of the rectum is not limited to the ultrasonic sensor 1. Any sensor except an ultrasonic sensor may be employed as long as the sensor can detect the width of the rectum. In the case of employing an ultrasonic sensor, the configuration of the ultrasonic sensor is not limited to the configuration described above. For example, an ultrasonic sensor including a plurality of transducers arranged in an array may be employed.

In the bowel movement prediction device 100, the ultrasonic sensor 1 and the device body 2 are integrally provided. However, the configuration is not limited to this example. For example, the ultrasonic sensor 1 and the device body 2 may be provided as separate components so that a subject wears only the ultrasonic sensor 1 and does not necessarily wear the device body 2. In this case, ultrasonic sensor 1 and the device body 2 communicate with each other by wire or wirelessly. A device worn by the subject includes at least the ultrasonic sensor 1 and may include other components. For example, the transmitter 3 and the receiver 4 may be separated from the device body 2 to be included in a device worn by the subject. In addition, the signal processor 51 may also be included in the device worn by the subject. Furthermore, the controller 5 and the memory 6 may be included in the device worn by the subject with only the notifier 7 and the input section 8 being separated from the device worn by the subject.

A device not worn by the subject may be constituted by a communication terminal such as a smartphone or a PC. For example, the ultrasonic sensor 1, the transmitter 3, the receiver 4, the controller 5, and the memory 6 may be integrally provided, and the notifier 7 and the input section 8 may be constituted by a communication terminal. In this case, the bowel movement prediction device 100 includes the ultrasonic sensor 1, the transmitter 3, the receiver 4, the controller 5, and the memory 6, and a communication terminal held by, for example, a caregiver functions as the notifier 7 and the input section 8. The bowel movement prediction device 100 determines a bowel movement timing as described above, and when the bowel movement timing arrives, the bowel movement prediction device 100 notifies the communication terminal of this arrival. In response to the notification of the bowel movement timing, the caregiver guides a person requiring care to the lavatory, and when actual bowel movement occurs, the caregiver transmits a report on this occurrence of actual bowel movement to the bowel movement prediction device 100 through the communication terminal. When the bowel movement prediction device 100 receives the report on the actual bowel movement, the bowel movement prediction device 100 modifies the determination threshold as described above.

A user may arbitrarily set a time interval defined as a margin immediately before bowel movement. For example, in a case where the user inputs, as a bowel movement timing, a time that is five minutes before bowel movement, the determiner 52 sets, as a determination threshold, a time difference between two reflected waves corresponding to a width of the rectum five minutes before bowel movement. The memory 6 stores a relationship between the time difference between two reflected waves and a bowel movement timing with reference to an average width of the rectum. The determiner 52 sets, as a determination threshold, the time difference between two reflected waves depending on an input time.

The notifier 7 is not limited to a vibrator. The notifier 7 may be an alarm, a lamp, or a combination thereof.

Modification of the determination threshold may not be based on an input from the input section 8 and may be based on the determination value (i.e., the width of the rectum). For example, in a case where determination of a bowel movement timing is repetitively performed at short intervals, determination values each for determining a time in which fecal matter is accumulated in the rectum to increase the width of the rectum, bowel movement occurs, and then the width of the rectum decreases are recorded on the memory 6 in detail. In such a case, it can be determined when actual bowel movement occurred, based on the determination values recorded on the memory 6. For example, actual bowel movement occurs at the time when the determination value (i.e., the width of the rectum) rapidly decreases from the maximum reached after issuance of a notification of arrival of a bowel movement timing. That is, the determination threshold may be modified based on the time from when the notification of the bowel movement timing is issued to when the determination value reaches the maximum. In this manner, in the configuration in which the determination threshold is modified based on the determination values, the determination threshold (i.e., the width of the rectum) at which the time to bowel movement is a predetermined set time can be obtained from actual data, and thus, the determination threshold can be more accurately modified.

The determination may be performed in such a manner that determination of a bowel movement timing is performed in relatively long cycle periods (e.g., at every 10 minutes) before arrival of the bowel movement timing and detection of the width of the rectum is performed in relatively short cycle periods (e.g., at every one minute) after determination of arrival of the bowel movement timing. In this case, the widths of the rectum before and after bowel movement can be more accurately detected and the determination threshold can be more accurately modified, with reduced power consumption.

In issuing a notification of arrival of a bowel movement timing, the determiner 52 may issue a notification of an approximate time before bowel movement. In this configuration, the determination threshold and a predicted time to bowel movement may also be modified based on actual bowel movement as described above.

The determiner 52 is not limited to a section that determines a bowel movement timing based on a time difference between two reflected waves, that is, the width of the rectum. For example, the determiner 52 may determine a bowel movement timing based on a rate of change of the time difference between two reflected waves, that is, a rate of change of the rectum width. That is, the detection of abrupt increase in the width of the rectum can be defined as arrival of fecal matter to the rectum, that is, arrival of a bowel movement timing.

In the above example, the transmitter 3 inputs a pulse signal to the ultrasonic sensor 1 as a driving signal. The driving signal, however, is not limited to a pulse signal. The driving signal may be a burst wave instead of a pulse wave.

A frequency modulated continuous wave may also be used as the driving signal. In this case, the determiner 52 analyzes the frequency of a received signal to detect the width of the rectum. A technique of the frequency analysis may be a fast Fourier transform (FFT) or a maximum entropy method (MEM).

In the above description, the determiner 52 determines a bowel movement timing by using only the time difference ΔT between the reflected wave from the anterior wall of the rectum and the reflected wave from the posterior wall of the rectum. The determination, however, is not limited to this example. For example, the determiner 52 may determine a bowel movement timing based on the state of fecal matter in addition to the time difference ΔT. Specifically, the determiner 52 may determine a bowel movement timing based on the time difference ΔT and a water content (or water proportion) of fecal matter detected from the received signal. More specifically, in the case of soft fecal matter, the bowel movement timing arrives relatively early. Thus, with a large water content, the determiner 52 modifies the bowel movement timing based on the time difference ΔT to a relatively earlier timing. If the water content is a predetermined level or higher, the determiner 52 may change the method of notification (e.g., increase the degree of vibrations), for example, in order to express urgency.

The water content of fecal matter can be detected based on a portion of the received signal between the peaks P5 and P6. Ultrasonic waves emitted to the inside of the body are also reflected on fecal matter to a small degree. The acoustic impedance of fecal matter also varies depending on the water content of fecal matter, and thus, the intensity of reflected waves from fecal matter also varies depending on the water content of fecal matter. For this reason, the determiner 52 can detect the water content of fecal matter based on a noise level of a portion of the received signal between the peaks P5 and P6. As the noise level increases, the water content of fecal matter decreases. As the noise level decreases, the water content of fecal matter increases. As another case, as the intensity of the reflected wave from fecal matter increases, ultrasonic waves that reach the posterior wall of the rectum decrease accordingly so that the reflected wave from the posterior wall of the rectum decreases. That is, the determiner 52 can also detect the water content of fecal matter based on an attenuation ratio of the reflected wave from the posterior wall of the rectum with respect to the reflected wave from the anterior wall of the rectum (i.e., amplitude of reflected wave from posterior wall of rectum/amplitude of reflected wave from anterior wall of rectum). As the attenuation ratio increases, the water content of fecal matter decreases, whereas as the attenuation ratio decreases, the water content of fecal matter increases.

That is, the ultrasonic sensor 1 functions not only as a sensor for detecting the width of the rectum but also as a sensor for detecting the state of fecal matter in the rectum. In step S5 described above, the determiner 52 may modify the determination threshold based on the state (i.e., water content) of fecal matter in determining a bowel movement timing from a comparison between the time difference ΔT and the determination threshold. Alternatively, the memory 6 may previously store a determination threshold in accordance with the water content of fecal matter so that the determiner 52 reads the determination threshold in accordance with the water content of fecal matter from the memory 6 to compare the time difference ΔT with this determination threshold.

In cases where a reflected wave has an attenuation ratio of approximately zero, that is, the reflected wave from the posterior wall of the rectum cannot be observed, the determiner 52 may determine that gas is accumulated in the rectum.

### <Detection of Bowel Sound>

The bowel movement prediction device may further include a bowel sound sensor that detects bowel sound to determine a bowel movement timing in consideration of bowel sound as well as the width of the rectum. FIG. 4 is a block diagram of a bowel movement prediction device 2100 including a bowel sound sensor. The bowel movement prediction device 2100 includes an ultrasonic unit 300, a bowel sound unit 400, and a communication terminal 200. The ultrasonic unit 300 and the communication terminal 200 of the bowel movement prediction device 2100 are the example described above in which the ultrasonic sensor and device body are provided as separate components and the communication terminal 200 functions as a device body.

Specifically, the ultrasonic unit 300 is disposed on a the skin of a subject at a location corresponding to the rectum (e.g., lower abdomen), transmits ultrasonic waves to the inside of the body, and receives reflected waves associated with the transmitted ultrasonic waves. The ultrasonic unit 300 includes an ultrasonic sensor 1, a first transmitter 303, a first receiver 304, a first controller 305, and a first communicator 306. The first transmitter 303 and the first receiver 304 correspond to the transmitter 3 and the receiver 4, respectively. The first controller 305 includes one or more processors and controls the ultrasonic unit 300. The first communicator 306 wirelessly communicates with the communication terminal 200.

The bowel sound unit 400 is disposed on the skin of the subject at a location corresponding to the large intestine (e.g., lower abdomen), and detects bowel sound in the body. The bowel sound unit 400 includes a microphone 401, a second receiver 404, a second controller 405, and a second communicator 406. The microphone 401 is an example of a sensor that detects bowel sound in the body. The second receiver 404 receives an electrical signal from the microphone 401. The receiver 404 includes an amplifier 441 and an A/D converter 442. The amplifier 441 amplifies a signal received from the microphone 401 and outputs the amplified signal to the A/D converter 442. The A/D converter 442 performs A/D conversion on the signal received from the amplifier 441, and outputs the resulting signal to the second controller 405. The second controller 405 includes one or more processors, and controls the bowel sound unit 400. The second communicator 406 wirelessly communicates with the communication terminal 200.

The communication terminal 200 includes a body-side controller 5, a memory 6, a notifier 7, an input section 8, and a body-side communicator 9. The body-side controller 5 and the body-side communicator 9 correspond to the controller 5 and the communicator 9, respectively. The communication terminal 200 includes a downloaded application dedicated to the bowel movement prediction device 2100 so that a bowel movement timing can be predicted by causing the application to operate. The communication terminal 200 is, for example, a smartphone or a PC. A CPU, a memory, a vibrator and a speaker, an input interface such as a touch panel, and a communicator unique to the communication terminal 200 function as the body-side controller 5, the memory 6, the notifier 7, the input section 8, and the body-side communicator 9, respectively. Through communication between the first communicator 306 and the body-side communicator 9, the communication terminal 200 and the ultrasonic unit 300 communicate with each other. Through communication between the second communicator 406 and the body-side communicator 9, the communication terminal 200 and the bowel sound unit 400 communicate with each other.

The communication terminal 200 transmits, to the ultrasonic unit 300, an instruction for causing the ultrasonic sensor 1 to transmit an ultrasonic wave in predetermined cycles. Here, the predetermined cycle refers to a cycle of determining arrival of a bowel movement timing.

When the ultrasonic unit 300 receives the instruction, the first controller 305 causes the first transmitter 303 to output a driving voltage to the ultrasonic sensor 1. Thereafter, when the first receiver 304 receives a signal received from the ultrasonic sensor 1, the first controller 305 transmits the received signal to the communication terminal 200 through the first communicator 306.

When the communication terminal 200 receives the signal received from the ultrasonic unit 300, the signal processor 51 performs signal processing on the received signal, and the determiner 52 determines a bowel movement timing by using the resulting signal. The processes of the signal processor 51 and the determiner 52 (specifically, signal processing, determination of arrival of a bowel movement timing, and control of the notifier 7 and other sections after the determination) are basically the same as those described above.

In addition to the processes described above, the communication terminal 200 receives the signal received from the bowel sound unit 400. Specifically, the bowel sound unit 400 continuously transmits, to the communication terminal 200, the signal received transmitted from the microphone 401 and received by the second receiver 404, through the second communicator 406.

When the communication terminal 200 receives the signal received from the bowel sound unit 400, the signal processor 51 performs signal processing on the received signal, and using the resulting signal, the determiner 52 detects mass peristalsis. Specifically, when peristalsis occurs in the large intestine, sound associated with the peristalsis is generated. The determiner 52 determines whether mass peristalsis occurs or not based on the level and/or the number of occurrences of sound in the intestine detected by using the microphone 401. For example, in a case where the level of bowel sound is a predetermined determination level or more, a case where the number of occurrences of bowel sound is a predetermined determination number or more, or a case where the level of bowel sound is a predetermined determination level or more and the number of occurrences of bowel sound is a predetermined determination number or more, the determiner 52 can determine that mass peristalsis occurs.

If mass peristalsis is detected, the determiner 52 makes the cycle of determination of arrival of a bowel movement timing (i.e., a cycle of issuing an instruction for causing the ultrasonic sensor 1 to transmit an ultrasonic wave) shorter than that before occurrence of the mass peristalsis. That is, the determiner 52 is configured to change the cycle of detecting the width of the rectum, specifically, extend the cycle before occurrence of mass peristalsis and shorten the cycle after occurrence of mass peristalsis. In this manner, while a small amount of fecal matter is accumulated in the rectum, power consumption is reduced, whereas when the amount of fecal matter accumulated in the rectum starts increases, the width of the rectum is detected in detail so that arrival of a bowel movement timing can be promptly determined without fail.

The cycle of determining arrival of a bowel movement timing may be changed based not only on the presence of mass peristalsis but also on the level and/or the number of occurrences of bowel sound caused by peristalsis. That is, bowel sound herein encompasses bowel sound caused by simple peristalsis as well as bowel sound caused by mass peristalsis. When peristalsis becomes active, fecal matter moves in the large intestine, and accumulation of fecal matter in the rectum is likely to start. Thus, when peristalsis becomes active, the cycle of determining a bowel movement timing may be made shorter than that when peristalsis is not active. In this case, the determination cycle may be changed not in two stages but in three or more stages or linearly (in such a manner that the cycle of determining arrival of a bowel movement timing becomes shorter as peristalsis becomes more active). In addition, the determiner 52 may change the mode of notification by the notifier 7 based on peristalsis. For example, in a case where the notifier 7 is a vibrator, the determiner 52 may intensify the mode of vibrations of the vibrator when the peristalsis becomes active. In issuing a notification, the determiner 52 may use only the vibrator when peristalsis is not active and use both the vibrator and an alarm when peristalsis is active.

In the foregoing description, the ultrasonic unit 300 and the bowel sound unit 400 are provided as separate units. Alternatively, the ultrasonic unit 300 and the bowel sound unit 400 may be integrally provided. As described above, the body-side controller 5, for example, may be integrated with the ultrasonic unit 300 and/or the bowel sound unit 400.

### DESCRIPTION OF REFERENCE CHARACTERS

- 100: bowel movement prediction device
- 1: ultrasonic sensor (sensor)
- 52: determiner
- 7: notifier
- 200: communication terminal

## Claims

1. A bowel movement prediction device comprising:
a sensor that detects a width of a rectum; and
a determiner that detects a bowel movement timing based on an output of the sensor.

2. The bowel movement prediction device of claim 1, further comprising
a notifier that issues a notification of arrival of a bowel movement timing, wherein
the determiner causes the notifier to operate when the determiner determines that a bowel movement timing arrives.

3. The bowel movement prediction device of claim 1, wherein
when the determiner determines that a bowel movement timing arrives, the determiner notifies an external device that the bowel movement timing arrives.

4. The bowel movement prediction device of any one of claims 1 to 3, wherein
the determiner is configured to determine that a bowel movement timing arrives if the width of the rectum or a rate of change of the width is greater than or equal to a predetermined determination threshold, and modify the determination threshold based on accepted feedback on actual bowel movement after determination of the bowel movement timing to .

5. The bowel movement prediction device of claim 4, wherein
the determiner is configured to accept an input indicating occurrence of actual bowel movement, and modify the determination threshold based on a time from when the determiner determines that the bowel movement timing arrives to when the determiner accepts the input indicating occurrence of actual bowel movement.

6. The bowel movement prediction device of claim 1, wherein
the sensor detects a state of fecal matter in the rectum in addition to the width of the rectum.

7. The bowel movement prediction device of claim 6, wherein
the determiner determines a bowel movement timing based on the width of the rectum and the state of fecal matter.

8. A bowel movement prediction method comprising the steps of:
detecting a width of a rectum; and
determining a bowel movement timing based on the detected width of the rectum.

9. The bowel movement prediction method of claim 8, further comprising the step of issuing a notification of arrival of a bowel movement timing when it is determined that the bowel movement timing arrives.

10. The bowel movement prediction method of claim 8, further comprising the step of modifying a determination accuracy in determining the bowel movement timing based on feedback on actual bowel movement, wherein
in the step of determining the bowel movement timing, it is determined that the bowel movement timing arrives if the width of the rectum or a rate of change of the width is greater than or equal to a predetermined determination threshold, and
in the step of modifying the determination accuracy, the feedback on actual bowel movement is accepted after determination of the bowel movement timing so that the determination threshold is modified.

11. The bowel movement prediction method of claim 10, wherein
in the step of modifying the determination accuracy, an input indicating occurrence of actual bowel movement is accepted, and the determination threshold is modified based on a time from when it is determined that the bowel movement timing arrives to when the input indicating occurrence of actual bowel movement is accepted.

12. The bowel movement prediction method of claim 8, further comprising the step of detecting a state of fecal matter in the rectum.

13. The bowel movement prediction method of claim 12, wherein
in the step of determining the bowel movement timing, the bowel movement timing is determined based on the detected width of the rectum and the detected state of fecal matter.

## Amended claims

### Amended claims under Art. 19.1 PCT

1. (original) A bowel movement prediction device comprising:
a sensor that detects a width of a rectum; and
a determiner that detects a bowel movement timing based on an output of the sensor.

2. (original) The bowel movement prediction device of claim 1, further comprising
a notifier that issues a notification of arrival of a bowel movement timing, wherein
the determiner causes the notifier to operate when the determiner determines that a bowel movement timing arrives.

3. (original) The bowel movement prediction device of claim 1, wherein
when the determiner determines that a bowel movement timing arrives, the determiner notifies an external device that the bowel movement timing arrives.

4. (original) The bowel movement prediction device of any one of claims 1 to 3, wherein
the determiner is configured to determine that a bowel movement timing arrives if the width of the rectum or a rate of change of the width is greater than or equal to a predetermined determination threshold, and modify the determination threshold based on accepted feedback on actual bowel movement after determination of the bowel movement timing to .

5. (original) The bowel movement prediction device of claim 4, wherein
the determiner is configured to accept an input indicating occurrence of actual bowel movement, and modify the determination threshold based on a time from when the determiner determines that the bowel movement timing arrives to when the determiner accepts the input indicating occurrence of actual bowel movement.

6. (original) The bowel movement prediction device of claim 1, wherein
the sensor detects a state of fecal matter in the rectum in addition to the width of the rectum.

7. (original) The bowel movement prediction device of claim 6, wherein
the determiner determines a bowel movement timing based on the width of the rectum and the state of fecal matter.

8. (original) A bowel movement prediction method comprising the steps of:
detecting a width of a rectum; and
determining a bowel movement timing based on the detected width of the rectum.

9. (original) The bowel movement prediction method of claim 8, further comprising the step of issuing a notification of arrival of a bowel movement timing when it is determined that the bowel movement timing arrives.

10. (original) The bowel movement prediction method of claim 8, further comprising the step of modifying a determination accuracy in determining the bowel movement timing based on feedback on actual bowel movement, wherein
in the step of determining the bowel movement timing, it is determined that the bowel movement timing arrives if the width of the rectum or a rate of change of the width is greater than or equal to a predetermined determination threshold, and
in the step of modifying the determination accuracy, the feedback on actual bowel movement is accepted after determination of the bowel movement timing so that the determination threshold is modified.

11. (original) The bowel movement prediction method of claim 10, wherein
in the step of modifying the determination accuracy, an input indicating occurrence of actual bowel movement is accepted, and the determination threshold is modified based on a time from when it is determined that the bowel movement timing arrives to when the input indicating occurrence of actual bowel movement is accepted.

12. (original) The bowel movement prediction method of claim 8, further comprising the step of detecting a state of fecal matter in the rectum.

13. (original) The bowel movement prediction method of claim 12, wherein
in the step of determining the bowel movement timing, the bowel movement timing is determined based on the detected width of the rectum and the detected state of fecal matter.

14. (new) The bowel movement prediction device of claim 1, further comprising
a communication terminal, wherein
the determination section transmits the determined bowel movement timing to the communication terminal.
